# EUROPEAN PATENT APPLICATION

(11) **EP 4 678 636 A1**
(43) Date of publication of application: **14.01.2026**
(21) Application number: 25713494.0
(22) Date of filing: 05.02.2025
(51) Int. Cl.: C07D 307/68, B01J 31/04, C07B 61/00, C07D 307/48

(54) **METHOD FOR PRODUCING FURAN-2,5-DICARBOXYLIC ACID**

(30) Priority: 09.05.2024 JP 2024076436
(71) Applicant: MITSUBISHI GAS CHEMICAL COMPANY, INC., Chiyoda-ku, Tokyo 100-8324 (JP)
(72) Inventor: WATANABE, Yuki, Tokyo 100-8324 (JP); MATSUURA, Yumi, Tokyo 100-8324 (JP); NAKAMURA, Goh, Tokyo 100-8324 (JP)
(74) Representative: Vossius & Partner Patentanwälte Rechtsanwälte mbB
(86) International application number: PCT/JP2025/003764
(87) International publication number: WO 2025/234174

(57) **Abstract**

There is provided a method for producing furan-2,5-dicarboxylic acid, including: an oxidation step 1 of continuously feeding 5-methylfurfural and an oxygen-containing gas in the presence of a lower aliphatic carboxylic acid, a bromine compound and a metal catalyst to a reaction container to carry out oxidation reaction under the following feeding condition 1 or the following feeding condition 2; and an oxidation step 2, after the oxidation step 1, of continuously feeding 5-methylfurfural and an oxygen-containing gas to the reaction container to carry out oxidation reaction under such a feeding condition that the oxygen concentration of the oxygen-containing gas and/or the molar ratio of oxygen in the oxygen-containing gas to 5-methylfurfural is higher than in the oxidation step 1. The feeding condition 1 is such that the oxygen concentration of the oxygen-containing gas is 10% by volume or higher and lower than 15% by volume, and the molar ratio of oxygen in the oxygen-containing gas to 5-methylfurfural is 2.5 to 4.2; and the feeding condition 2 is such that the oxygen concentration of the oxygen-containing gas is 15 to 30% by volume, and the molar ratio of oxygen in the oxygen-containing gas to 5-methylfurfural is 1.0 or higher and lower than 2.5.

## Description

### Technical Field

The present invention relates to a method for producing furan-2,5-dicarboxylic acid.

### Background Art

Furan-2,5-dicarboxylic acid (FDCA) is a very useful compound as a starting material or an intermediate for resins such as polyethylene furanoate, paints, fibers and the like.

Furan-2,5-dicarboxylic acid, in particular, can be synthesized from a furfural compound derived from cellulose, which is a biomass raw material, and it is therefore useful as a biomass-derived raw material for resins and a biomass-derived raw material for fibers to replace fossil raw materials.

Examples of the furfural compound include 5-substituted furfural such as 5-hydroxymethylfurfural and 5-methylfurfural. Among these, 5-methylfurfural can be produced also from non-edible raw materials such as cellulose, unlike 5-hydroxymethylfurfural, which is produced from saccharides such as glucose and fructose, i.e., from edible raw materials, and it is therefore anticipated that 5-methylfurfural will serve as a biomass-derived raw material which can reduce the environmental impact.

As a method for producing furan-2,5-dicarboxylic acid, a method including oxidizing a 5-substituted furfural has been carried out. Particularly, a production method involving liquid-phase oxidation with an oxygen-containing gas such as air has been developed.

For example, PTL 1 discloses a method including oxidizing 5-hydroxymethylfurfural or an ester or the like thereof to furan-2,5-dicarboxylic acid in the presence of a cobalt- and manganese-based oxidation catalyst containing bromine at 140 to 200°C at an oxygen partial pressure of 1 to 10 bars.

PTL 2 discloses a method for producing furan-2,5-dicarboxylic acid, the method including oxidizing a furan composition containing 5-(acetoxymethyl)-2-furoic acid or the like as a first compound and a 5-substituted furfural or the like as a second compound in the presence of a catalyst, and this method is disclosed as a method aiming at improvement in the selectivity and the yield of FDCA.

Then, PTL 3 discloses a method for producing 2,5-furandicarboxylic acid, the method including feeding 5-methylfurfural, an oxidizing gas and further an alkoxymethyl-2,5-furfural, to subject these to oxidation at 150 to 210°C in the presence of a catalyst containing cobalt, manganese and bromine, and acetic acid, and separating a solid 2,5-furandicarboxylic acid from a crude product, and this method is disclosed as a method aiming to obtain FDCA in a high yield.

### Citation List

### Patent Literature

PTL1: JP 2013-507359 A
PTL2: US 9321744 B
PTL3: WO 2023/242152

### Summary of Invention

### Technical Problem

PTLs 1 and 2 each describe a production method of FDCA in which 5-methylfurfural is used as a starting material. However, the FDCA yield remains as low as 39.94 to 46.25%.

Then, PTL3 describes a production method of FDCA in which a mixture of an alkoxymethylfurfural and 5-methylfurfural are used as a starting material, and in Comparative Examples 2 to 4 therein, a production method is described in which only 5-methylfurfural is used as a starting material with air diluted to an oxygen concentration of 8% as an oxidant (oxidizing gas). In the case of using the gas with an oxygen concentration of 8% as a main oxidizing gas, however, there are many industrial problems including costs for the gas recovery and nitrogen used for lowering the oxygen concentration in the industrial production. Further, there is no description about the amount of the air relative to the furfural compound as a substrate, and in the case of carrying out the reaction under the condition described in PTL3 and using only air, which is industrially advantageous, a rise in the oxygen concentration of off-gas cannot be suppressed. Further even if the amount of the air to be fed is lowered in order to suppress the rise in the oxygen concentration of the off-gas by raising the oxygen conversion rate, there arises the problem of deteriorating the reaction yield.

Hence, there is a need for a method for industrially safely obtaining furan-2,5-dicarboxylic acid in a high yield.

Then, an object of the present invention is to provide a method for producing furan-2,5-dicarboxylic acid that can provide furan-2,5-dicarboxylic acid in a high yield and is also excellent in the conversion rate and industrially highly safe.

### Solution to Problem

As a result of exhaustive studies, the present inventors have found that by adopting a method comprising two oxidation steps in which 5-methylfurfural and an oxygen-containing gas are fed under specific conditions, the above-mentioned object can be achieved.

Specifically, the present invention relates to the following.
[1] A method for producing furan-2,5-dicarboxylic acid comprising:
   an oxidation step 1 of continuously feeding 5-methylfurfural and an oxygen-containing gas in the presence of a lower aliphatic carboxylic acid, a bromine compound and a metal catalyst to a reaction container to carry out oxidation reaction under the following feeding condition 1 or the following feeding condition 2, and
   an oxidation step 2, after the oxidation step 1, of continuously feeding 5-methylfurfural and an oxygen-containing gas to the reaction container to carry out oxidation reaction under such a feeding condition that at least one of the oxygen concentration of the oxygen-containing gas and the molar ratio of oxygen in the oxygen-containing gas to 5-methylfurfural is higher than in the oxidation step 1:
      the feeding condition 1: the oxygen concentration of the oxygen-containing gas is 10% by volume or higher and lower than 15% by volume, and the molar ratio of oxygen in the oxygen-containing gas to 5-methylfurfural is 2.5 to 4.2; and
      the feeding condition 2: the oxygen concentration of the oxygen-containing gas is 15 to 30% by volume, and the molar ratio of oxygen in the oxygen-containing gas to 5-methylfurfural is 1.0 or higher and lower than 2.5.
[1a] A method for producing furan-2,5-dicarboxylic acid comprising:
   an oxidation step 1 of continuously feeding 5-methylfurfural and an oxygen-containing gas in the presence of a lower aliphatic carboxylic acid, a bromine compound and a metal catalyst to a reaction container to carry out oxidation reaction under the following feeding condition 1 or the following feeding condition 2; wherein the bromine compound is at least one selected from the group consisting of hydrogen bromide and bromide salts, and the metal catalyst is at least one selected from the group consisting of cobalt catalysts and manganese catalysts, and
   an oxidation step 2, after the oxidation step 1, of continuously feeding 5-methylfurfural and an oxygen-containing gas to the reaction container to carry out oxidation reaction under such a feeding condition that at least one of the oxygen concentration of the oxygen-containing gas and the molar ratio of oxygen in the oxygen-containing gas to 5-methylfurfural is higher than in the oxidation step 1:
      the feeding condition 1: the oxygen concentration of the oxygen-containing gas is 10% by volume or higher and lower than 15% by volume, and the molar ratio of oxygen in the oxygen-containing gas to 5-methylfurfural is 2.5 to 4.2; and
      the feeding condition 2: the oxygen concentration of the oxygen-containing gas is 15 to 30% by volume, and the molar ratio of oxygen in the oxygen-containing gas to 5-methylfurfural is 1.0 or higher and lower than 2.5.
[2] The method for producing furan-2,5-dicarboxylic acid according to the above [1] or [1a], wherein
   the feeding condition in the oxidation step 1 is the feeding condition 1, and the oxygen concentration of the oxygen-containing gas in the oxidation step 2 is higher by 5% by volume or higher than the oxygen concentration of the oxygen-containing gas in the oxidation step 1; or
   the feeding condition in the oxidation step 1 is the feeding condition 2, and the molar ratio of oxygen in the oxygen-containing gas to 5-methylfurfural in the oxidation step 2 is higher by 1.5 or higher than the molar ratio of oxygen in the oxygen-containing gas to 5-methylfurfural in the oxidation step 1.
[3] The method for producing furan-2,5-dicarboxylic acid according to the above [1], [1a] or [2], wherein the feeding condition of 5-methylfurfural and the oxygen-containing gas in the oxidation step 2 is the following feeding condition 3:
   the feeding condition 3: the oxygen concentration of the oxygen-containing gas is 15 to 30% by volume, and the molar ratio of oxygen in the oxygen-containing gas to 5-methylfurfural is 2.5 to 4.2.
[4] The method for producing furan-2,5-dicarboxylic acid according to any one of the above [1], [1a], [2] and [3], wherein the feeding condition of 5-methylfurfural and the oxygen-containing gas in the oxidation step 1 is the following feeding condition 4:
   the feeding condition 4: the product of the oxygen concentration (volume) of the oxygen-containing gas and the molar ratio of oxygen in the oxygen-containing gas to 5-methylfurfural is 0.3 to 0.4.
[5] The method for producing furan-2,5-dicarboxylic acid according to any one of the above [1], [1a], and [2] to [4], wherein the oxygen partial pressure in feeding of the oxygen-containing gas in the oxidation step 1 is 0.05 to 0.2 MPa.
[6] The method for producing furan-2,5-dicarboxylic acid according to any one of the above [1], [1a], and [2] to [5], wherein the reaction temperature in the oxidation step 1 is 130 to 180°C.
[7] The method for producing furan-2,5-dicarboxylic acid according to any one of the above [1], [1a], and [2] to [6], wherein the reaction temperature and the oxygen partial pressure in feeding of the oxygen-containing gas in the oxidation step 1 are under the following condition 5 or the following condition 6:
   the condition 5: the reaction temperature is 130°C or higher and lower than 160°C, and the oxygen partial pressure in feeding of the oxygen-containing gas is 0.10 to 0.2 MPa; and
   the condition 6: the reaction temperature is 160 to 180°C, and the oxygen partial pressure in feeding of the oxygen-containing gas is 0.05 MPa or higher and lower than 0.10 MPa.
[8] The method for producing furan-2,5-dicarboxylic acid according to any one of the above [1], [1a], and [2] to [7], wherein the oxygen partial pressure in feeding of the oxygen-containing gas in the oxidation step 2 is 0.05 to 0.2 MPa.
[9] The method for producing furan-2,5-dicarboxylic acid according to any one of the above [1], [1a], and [2] to [8], wherein the reaction temperature in the oxidation step 2 is 130 to 180°C.
[10] The method for producing furan-2,5-dicarboxylic acid according to any one of the above [1], [1a], and [2] to [9], wherein the reaction temperature and the oxygen partial pressure in feeding of the oxygen-containing gas in the oxidation step 2 are under the following condition 7 or the following condition 8:
   the condition 7: the reaction temperature is 130°C or higher and lower than 160°C, and the oxygen partial pressure in feeding of the oxygen-containing gas is 0.15 to 0.2 MPa; and
   the condition 8: the reaction temperature is 160 to 180°C, and the oxygen partial pressure in feeding of the oxygen-containing gas is 0.11 MPa or higher and lower than 0.15 MPa.
   [11] The method for producing furan-2,5-dicarboxylic acid according to any one of the above [1], [1a], and [2] to [10], wherein the reaction time in the oxidation step 1 is 3 to 200 min.
[12] The method for producing furan-2,5-dicarboxylic acid according to any one of the above [1], [1a], and [2] to [11], wherein the metal catalyst is at least one selected from the group consisting of aliphatic carboxylates of cobalt and aliphatic carboxylates of manganese.
[13] The method for producing furan-2,5-dicarboxylic acid according to any one of the above [1], [1a], and [2] to [12], wherein the lower aliphatic carboxylic acid is acetic acid.
[14] The method for producing furan-2,5-dicarboxylic acid according to any one of the above [1], [1a], and [2] to [13], wherein the bromine compound is at least one selected from the group consisting of hydrogen bromide, sodium bromide, potassium bromide and ammonium bromide.
[15] The method for producing furan-2,5-dicarboxylic acid according to any one of the above [1], [1a], and [2] to [14], wherein a gas other than oxygen contained in the oxygen-containing gas in the oxidation step 1 contains 95% by volume or higher of nitrogen.
[16] The method for producing furan-2,5-dicarboxylic acid according to any one of the above [1], [1a], and [2] to [15], wherein a gas other than oxygen contained in the oxygen-containing gas in the oxidation step 2 contains 95% by volume or higher of nitrogen.

### Advantageous Effects of Invention

According to the present invention, there can be provided a method for producing furan-2,5-dicarboxylic acid that can provide furan-2,5-dicarboxylic acid in a high yield and also is excellent in the conversion rate and industrially highly safe.

### Description of Embodiments

A method for producing furan-2,5-dicarboxylic acid according to the present invention comprises:
an oxidation step 1 of continuously feeding 5-methylfurfural and an oxygen-containing gas in the presence of a lower aliphatic carboxylic acid, a bromine compound and a metal catalyst to a reaction container to carry out oxidation reaction under the following feeding condition 1 or the following feeding condition 2, and
an oxidation step 2, after the oxidation step 1, of continuously feeding 5-methylfurfural and an oxygen-containing gas to the reaction container to carry out oxidation reaction under such a feeding condition that at least one of the oxygen concentration of the oxygen-containing gas and the molar ratio of oxygen in the oxygen-containing gas to 5-methylfurfural is higher than in the oxidation step 1:
   the feeding condition 1: the oxygen concentration of the oxygen-containing gas is 10% by volume or higher and lower than 15% by volume, and the molar ratio of oxygen in the oxygen-containing gas to 5-methylfurfural is 2.5 to 4.2; and
   the feeding condition 2: the oxygen concentration of the oxygen-containing gas is 15 to 30% by volume, and the molar ratio of oxygen in the oxygen-containing gas to 5-methylfurfural is 1.0 or higher and lower than 2.5.

Then, a method for producing furan-2,5-dicarboxylic acid according to the present invention preferably comprises:
an oxidation step 1 of continuously feeding 5-methylfurfural and an oxygen-containing gas in the presence of a lower aliphatic carboxylic acid, a bromine compound and a metal catalyst to a reaction container to carry out oxidation reaction under the following feeding condition 1 or the following feeding condition 2; wherein the bromine compound is at least one selected from the group consisting of hydrogen bromide and bromide salts, and the metal catalyst is at least one selected from the group consisting of cobalt catalysts and manganese catalysts, and
an oxidation step 2, after the oxidation step 1, of continuously feeding 5-methylfurfural and an oxygen-containing gas to the reaction container to carry out oxidation reaction under such a feeding condition that at least one of the oxygen concentration of the oxygen-containing gas and the molar ratio of oxygen in the oxygen-containing gas to 5-methylfurfural is higher than in the oxidation step 1:
   the feeding condition 1: the oxygen concentration of the oxygen-containing gas is 10% by volume or higher and lower than 15% by volume, and the molar ratio of oxygen in the oxygen-containing gas to 5-methylfurfural is 2.5 to 4.2; and
   the feeding condition 2: the oxygen concentration of the oxygen-containing gas is 15 to 30% by volume, and the molar ratio of oxygen in the oxygen-containing gas to 5-methylfurfural is 1.0 or higher and lower than 2.5. The production method of the present invention will be described in detail below.

### [Oxidation step 1]

In the method for producing furan-2,5-dicarboxylic acid of the present invention, first, the oxidation step 1 is carried out in which 5-methylfurfural and an oxygen-containing gas are continuously fed in the presence of a lower aliphatic carboxylic acid, a bromine compound and a metal catalyst to a reaction container to carry out oxidation reaction under the following feeding condition 1 or the following feeding condition 2:
the feeding condition 1: the oxygen concentration of the oxygen-containing gas is 10% by volume or higher and lower than 15% by volume, and the molar ratio of oxygen in the oxygen-containing gas to 5-methylfurfural is 2.5 to 4.2; and
the feeding condition 2: the oxygen concentration of the oxygen-containing gas is 15 to 30% by volume, and the molar ratio of oxygen in the oxygen-containing gas to 5-methylfurfural is 1.0 or higher and lower than 2.5.

It is preferable that the lower aliphatic carboxylic acid, the bromine compound and the metal catalyst be accommodated in advance in the reaction container before 5-methylfurfural and the oxygen-containing gas are fed, and it is preferable that 5-methylfurfural and the oxygen-containing gas be continuously fed to the reaction container accommodating the lower aliphatic carboxylic acid, the bromine compound and the metal catalyst under the feeding condition 1 or the feeding condition 2.

The amounts of the lower aliphatic carboxylic acid, the bromine compound and the metal catalyst may be appropriately adjusted according to the shape, the volume and others of the reaction container. In view of smoothly initiating the oxidation reaction in the present step, it is preferable that the amounts be such that these can be mixed sufficiently with 5-methylfurfural and the oxygen-containing gas and such that the initial reaction temperature can be adjusted to a target temperature.

Alternatively, it is preferable that the lower aliphatic carboxylic acid, the bromine compound and the metal catalyst be mixed with 5-methylfurfural to be fed in the present step, before feeding. That is, it is preferable that 5-methylfurfural, the lower aliphatic carboxylic acid, the bromine compound, the metal catalyst and the oxygen-containing gas be continuously fed under the feeding condition 1 or the feeding condition 2.

Further, also in the oxidation step 2 to be carried out after the oxidation step 1, it is preferable that the lower aliphatic carboxylic acid, the bromine compound and the metal catalyst be mixed with 5-methylfurfural to be continuously fed. That is, it is preferable that the oxidation step 2 be a step, after the oxidation step 1, of continuously feeding 5-methylfurfural, the lower aliphatic carboxylic acid, the bromine compound, the metal catalyst and the oxygen-containing gas to the reaction container to carry out oxidation reaction.

It is preferable that the amounts of the lower aliphatic carboxylic acid, the bromine compound and the metal catalyst be determined with respect to the whole 5-methylfurfural to be used in the present production method; and in the case of carrying out the oxidation step 2 continuously for a long period, it is preferable that components shown below be mixed with 5-methylfurfural before feeding to the reaction container such that the ratio (amount) of each component to the 5-methylfurfural to be continuously fed satisfies the preferable ratio (amount) of each component, which will be shown below.

### <Lower aliphatic carboxylic acid>

The lower aliphatic carboxylic acid to be used in the present production method is preferably an aliphatic carboxylic acid having 1 to 4 carbon atoms, more preferably an aliphatic carboxylic acid having 2 or 3 carbon atoms and still more preferably an aliphatic carboxylic acid having 2 carbon atoms.

Specifically, the lower aliphatic carboxylic acid is preferably at least one selected from the group consisting of formic acid, acetic acid, propionic acid and butyric acid, more preferably at least one selected from the group consisting of acetic acid and propionic acid and still more preferably acetic acid. In the case of using acetic acid, acetic acid may be mixed with water described later to prepare a mixed liquid, which may be used, or acetic acid alone may be used. As the acetic acid, aqueous acetic acid, which is a mixed liquid of water and acetic acid, is preferably used in view of making it easy for the bromine compound and the metal catalyst to be dissolved.

Use of the above lower aliphatic carboxylic acid is preferable because the activity of the catalyst can be enhanced.

The amount of the lower aliphatic carboxylic acid to be used in the present production method is preferably 100 to 1,500 parts by mass, more preferably 200 to 800 parts by mass, still more preferably 300 to 700 parts by mass and further still more preferably 400 to 600 parts by mass, per 100 parts by mass of the whole 5-methylfurfural.

When the amount of the lower aliphatic carboxylic acid is in the above range, the viscosity of the reaction system can be suitably adjusted in the present step and the subsequent oxidation step 2, to thereby result in ease in handling. Further, control of the reaction heat is also made possible.

Here, "the whole 5-methylfurfural" refers to "the whole of 5-methylfurfural to be introduced to the reaction container from the oxidation step 1 to the end of the oxidation step 2 and used in oxidation reaction". The same applies hereinafter.

The lower aliphatic carboxylic acids may be used singly, or two or more thereof may be used.

### <Bromine compound>

Preferable examples of the bromine compound to be used in the present production method includes hydrogen bromide, bromide salts and organic bromine compounds. The bromine compound is more preferably at least one selected from the group consisting of hydrogen bromide and bromide salts, and is still more preferably hydrogen bromide.

Hydrogen bromide is preferably used in a form of an aqueous solution.

Specific examples of the bromide salts include sodium bromide, potassium bromide and ammonium bromide.

The amount of the bromine compound to be used in the present production method is, in terms of bromine, preferably 1.0 to 6.0 parts by mass, more preferably 1.5 to 5.5 parts by mass, still more preferably 2.0 to 4.5 parts by mass and further still more preferably 3.0 to 3.5 parts by mass, per 100 parts by mass of the whole 5-methylfurfural.

The amount of the bromine compound in the above range is preferable for the following reasons: corrosion of the reaction container and the like is suppressed and simultaneously, the reaction velocity and the yield in the present step and the subsequent oxidation step 2 are improved.

The bromine compounds may be used singly, or two or more thereof may be used.

### <Metal catalyst>

The metal catalyst to be used in the present production method is preferably at least one selected from the group consisting of transition metal catalysts and rare earth metal catalysts and more preferably a transition metal catalyst.

Specifically, the transition metal catalyst is preferably at least one selected from the group consisting of cobalt catalysts, manganese catalysts, zirconium catalysts, nickel catalysts and cesium catalysts, and more preferably at least one selected from the group consisting of cobalt catalysts and manganese catalysts. Use of both of a cobalt catalyst and a manganese catalyst is still more preferable.

As described above, the metal catalyst to be used in the present step is preferably at least one selected from the group consisting of cobalt catalysts, manganese catalysts, zirconium catalysts, nickel catalysts and cesium catalysts, and more preferably at least one selected from the group consisting of cobalt catalysts and manganese catalysts. Use of both of a cobalt catalyst and a manganese catalyst is still more preferable.

Although the metal catalyst can be used in, for example, a form of a salt, a simple metal, an oxide, or a hydroxide, the metal catalyst to be used in the present step is preferably a salt, more preferably an aliphatic carboxylate salt, still more preferably a lower aliphatic carboxylate salt and further still more preferably an acetate salt. Above all, further still more preferable is at least one selected from the group consisting of cobalt acetate and manganese acetate.

Use of the above metal catalyst is preferable because furan-2,5-dicarboxylic acid can be obtained in a high yield.

The amount of the metal catalyst to be used in the present production method is, in terms of metal element, preferably 0.05 to 4.0 parts by mass, more preferably 0.1 to 3.5 parts by mass, still more preferably 0.15 to 3.0 parts by mass and further still more preferably 0.2 to 2.5 parts by mass, per 100 parts by mass of the whole 5-methylfurfural to be used as a starting material in the oxidation reaction.

The amount of the metal catalyst in the above range is preferable for the following reasons: while suppressing side reactions, the reaction velocity in the present step and the subsequent oxidation step 2 is improved and the yield is improved.

When the catalyst concentration is the above-mentioned lower limit value or higher, the reaction velocity is improved and the yield is also improved. When the catalyst concentration is the above-mentioned upper limit value or lower, the catalyst cost is low and no adverse effect on the reaction is caused.

The metal catalysts may be used singly, or two or more thereof may be used.

It is not clear why furan-2,5-dicarboxylic acid is obtained in an excellent conversion rate and a high yield by the production method comprising the present oxidation step 1 involving use of 5-methylfurfural as a starting material, and it is also not clear why the highly safe production in an industrial point of view can be carried out by the production method comprising the present oxidation step 1. However, the reason therefor is conceivably as follows.

It is conceivable that part of 5-methylfurfural becomes active species by oxidation of 5-methylfurfural by the present oxidation step 1 under a specific mild condition, and that the oxidation reaction in the subsequent oxidation step 2 can be thus progressed smoothly. It is conceivable that hence, no stagnation of oxygen is caused, and that the oxygen concentration in a discharged gas (off-gas) can be kept low, enabling highly safe production. Further, it is conceivable that the starting material is evenly consumed during the reaction, resulting in suppression of side reactions, an excellent conversion rate, and an improved yield.

### <Water>

In the production method of the present invention, water may be used. In view of ease in dissolving the bromine compound, use of water is preferable.

The amount of water to be used in the present production method is preferably 10 to 200 parts by mass, more preferably 10 to 100 parts by mass, still more preferably 10 to 50 parts by mass and further still more preferably 10 to 40 parts by mass, per 100 parts by mass of the whole 5-methylfurfural to be used as a starting material in the oxidation reaction.

When the water concentration is in the above range, the bromine compound can be dissolved while the deterioration of the catalyst activity is prevented. This can improve the yield.

### <Oxygen-containing gas>

The oxygen-containing gas to be used in the present step is a mixed gas of oxygen and a gas other than oxygen. The gas other than oxygen to be mixed with oxygen is preferably an inert gas and more preferably nitrogen. The gas other than oxygen to be contained in the oxygen-containing gas in the oxidation step 1 is preferably a gas containing 95% by volume or higher of nitrogen and preferably a gas containing 98% by volume or higher of nitrogen.

As a gas containing nitrogen and oxygen, air is preferable.

Further, in order to adjust the oxygen concentration, oxygen gas or an oxygen-containing gas having a high oxygen concentration may be mixed with an inert gas such as nitrogen, before use.

For example, the gas having an oxygen concentration of 10% by volume or higher and lower than 15% by volume in the feeding condition 1 of the present step can be obtained by mixing, with nitrogen, air having an oxygen concentration of 20.9% by volume. Then, as the gas having an oxygen concentration of 15 to 30% by volume in the feeding condition 2 of the present step, air having an oxygen concentration of 20.9% by volume can be used as it is. In the present step, it is thus preferable that the oxygen concentration be adjusted by air and nitrogen to obtain the oxygen-containing gas. From the safety and economical viewpoint, it is preferable to use air or a mixed gas of air and nitrogen as the oxygen-containing gas.

### <Condition and others of the oxidation step 1>

The present step (oxidation step 1) involves continuously feeding 5-methylfurfural and the oxygen-containing gas to the reaction container to carry out oxidation reaction under the above-mentioned feeding condition 1 or the above-mentioned feeding condition 2.

Here, "continuously feeding 5-methylfurfural and the oxygen-containing gas" means that the oxidation reaction of 5-methylfurfural in the reaction container and the feeding of 5-methylfurfural and the oxygen-containing gas are parallelly carried out, and that 5-methylfurfural and the oxygen-containing gas are fed for at least time longer than 50% of the reaction time during the reaction time from the initiation of the oxidation reaction to the end of the oxidation reaction. The feeding of 5-methylfurfural is carried out preferably for 60% or longer of the reaction time in the oxidation step 1, more preferably for 80% or longer thereof and still more preferably for 90% or longer thereof, and may be carried out for 100% of the reaction time in the oxidation step 1. The feeding of the oxygen-containing gas is carried out preferably for 60% or longer of the reaction time in the oxidation step 1, more preferably for 80% or longer thereof and still more preferably for 90% or longer thereof, and may be carried out for 100% of the reaction time in the oxidation step 1.

The feeding condition 1 and the feeding condition 2 are suitable as conditions to cause oxidation reaction under relatively mild conditions. It is conceivable that by carrying out the oxidation reaction under such conditions, part of 5-methylfurfural can become active species, and that the oxidation reaction in the subsequent oxidation step 2 can be thus progressed smoothly.

The feeding condition 1 is as follows: the oxygen concentration of the oxygen-containing gas is 10% by volume or higher and lower than 15% by volume, and the molar ratio of oxygen in the oxygen-containing gas to 5-methylfurfural is 2.5 to 4.2.

In the case of feeding 5-methylfurfural and the oxygen-containing gas under the feeding condition 1, the oxygen concentration of the oxygen-containing gas to be used is 10% by volume or higher and lower than 15% by volume, and preferably 10 to 14% by volume, more preferably 10 to 13% by volume and still more preferably 11 to 13% by volume. Due to the oxygen concentration of the oxygen-containing gas in the above range, furan-2,5-dicarboxylic acid can be industrially highly safely obtained in a high conversion rate and a high yield.

In the case of feeding 5-methylfurfural and the oxygen-containing gas under the feeding condition 1, the molar ratio of oxygen in the oxygen-containing gas to 5-methylfurfural is 2.5 to 4.2, and preferably 3.0 to 4.0, more preferably 3.1 to 3.8 and still more preferably 3.2 to 3.5. Due to the molar ratio of oxygen in the oxygen-containing gas in the above range, furan-2,5-dicarboxylic acid can be industrially highly safely obtained in a high conversion rate and a high yield.

It is conceivable that the feeding condition 1, in which the oxygen concentration is rather low, can industrially safely prepare active species for the subsequent oxidation reaction.

The feeding condition 2 is as follows: the oxygen concentration of the oxygen-containing gas is 15 to 30% by volume, and the molar ratio of oxygen in the oxygen-containing gas to 5-methylfurfural is 1.0 or higher and lower than 2.5.

In the case of feeding 5-methylfurfural and the oxygen-containing gas under the feeding condition 2, the oxygen concentration of the oxygen-containing gas to be used is 15 to 30% by volume, and preferably 18 to 28% by volume, more preferably 20 to 25% by volume and still more preferably 20 to 22% by volume. Due to the oxygen concentration of the oxygen-containing gas in the above range, furan-2,5-dicarboxylic acid can be industrially highly safely obtained in a high conversion rate and a high yield.

In the case of feeding 5-methylfurfural and the oxygen-containing gas under the feeding condition 2, the molar ratio of oxygen in the oxygen-containing gas to 5-methylfurfural is 1.0 or higher and lower than 2.5, and preferably 1.0 to 2.3, more preferably 1.3 to 2.1 and still more preferably 1.5 to 2.0. Due to the molar ratio of oxygen in the oxygen-containing gas to 5-methylfurfural in the above range, furan-2,5-dicarboxylic acid can be industrially highly safely obtained in a high conversion rate and a high yield.

It is conceivable that the feeding condition 2, in which the molar ratio of oxygen in the oxygen-containing gas to 5-methylfurfural is rather low, can industrially safely prepare active species for the subsequent oxidation reaction.

In the oxidation step 1, 5-methylfurfural and the oxygen-containing gas are fed under the feeding condition 1 or the feeding condition 2. In either feeding condition case, the feeding condition of 5-methylfurfural and the oxygen-containing gas in the oxidation step 1 is preferably the following feeding condition 4.

The feeding condition 4: the product of the oxygen concentration (volume) of the oxygen-containing gas and the molar ratio of oxygen in the oxygen-containing gas to 5-methylfurfural is 0.3 to 0.5.

The product of the oxygen concentration (volume) of the oxygen-containing gas and the molar ratio of oxygen in the oxygen-containing gas to 5-methylfurfural is preferably 0.3 to 0.5, more preferably 0.35 to 0.45, still more preferably 0.37 to 0.45 and further still more preferably 0.40 to 0.44. Due to the product of the oxygen concentration (volume) of the oxygen-containing gas and the molar ratio of oxygen in the oxygen-containing gas to 5-methylfurfural in the above range, furan-2,5-dicarboxylic acid can be industrially highly safely obtained in a high conversion rate and a high yield.

In the oxidation step 1, 5-methylfurfural and the oxygen-containing gas are fed under the feeding condition 1 or the feeding condition 2, and in either feeding condition case, the oxygen partial pressure in feeding of the oxygen-containing gas in the oxidation step 1 is preferably 0.05 to 0.2 MPa.

In the oxidation step 1, 5-methylfurfural and the oxygen-containing gas are fed under the feeding condition 1 or the feeding condition 2, and in either feeding condition case, the reaction temperature in the oxidation step 1 is preferably 130 to 180°C.

Then, there is a preferable combination of the oxygen partial pressure and the reaction temperature. The preferable combination is specifically shown below.

In the oxidation step 1, 5-methylfurfural and the oxygen-containing gas are fed under the feeding condition 1 or the feeding condition 2, and in either feeding condition case, the reaction temperature and the oxygen partial pressure in feeding of the oxygen-containing gas in the oxidation step 1 are preferably under the following condition 5 or the following condition 6.

The condition 5: the reaction temperature is 130°C or higher and lower than 160°C, and the oxygen partial pressure in feeding of the oxygen-containing gas is 0.10 to 0.2 MPa.

The condition 6: the reaction temperature is 160 to 180°C, and the oxygen partial pressure in feeding of the oxygen-containing gas is 0.05 MPa or higher and lower than 0.10 MPa.

The condition 5 is as follows: the reaction temperature is 130°C or higher and lower than 160°C, and the oxygen partial pressure in feeding of the oxygen-containing gas is 0.10 to 0.2 MPa.

In the case of feeding 5-methylfurfural and the oxygen-containing gas under the condition 5, the reaction temperature is preferably 130°C or higher and lower than 160°C, more preferably 135 to 155°C and still more preferably 140 to 150°C. Due to the reaction temperature in the above range, furan-2,5-dicarboxylic acid can be industrially highly safely obtained in a high conversion rate and a high yield.

In the case of feeding 5-methylfurfural and the oxygen-containing gas under the condition 5, the oxygen partial pressure in feeding of oxygen-containing gas is preferably 0.10 to 0.2 MPa, more preferably 0.12 to 0.2 MPa and still more preferably 0.15 to 0.2 MPa. Due to the oxygen partial pressure in the above range, furan-2,5-dicarboxylic acid can be industrially highly safely obtained in a high conversion rate and a high yield.

It is conceivable that the condition 5, in which the reaction temperature is rather low, can industrially safely prepare active species for the subsequent oxidation reaction.

The condition 6 is as follows: the reaction temperature is 160 to 180°C, and the oxygen partial pressure in feeding of the oxygen-containing gas is 0.05 MPa or higher and lower than 0.10 MPa.

In the case of feeding 5-methylfurfural and the oxygen-containing gas under the condition 6, the reaction temperature is preferably 160 to 180°C, more preferably 165 to 180°C and still more preferably 170 to 175°C. Due to the reaction temperature in the above range, furan-2,5-dicarboxylic acid can be industrially highly safely obtained in a high conversion rate and a high yield.

In the case of feeding 5-methylfurfural and the oxygen-containing gas under the condition 6, the oxygen partial pressure in feeding of the oxygen-containing gas is preferably 0.05 MPa or higher and lower than 0.10 MPa, more preferably 0.05 to 0.09 MPa and still more preferably 0.05 to 0.08 MPa. Due to the oxygen partial pressure in the above range, furan-2,5-dicarboxylic acid can be industrially highly safely obtained in a high conversion rate and a high yield.

It is conceivable that the condition 6, in which the oxygen partial pressure is rather low, can industrially safely prepare active species for the subsequent oxidation reaction.

The reaction time in the oxidation step 1 is not especially limited, and is preferably 3 to 200 min, more preferably 3 to 100 min, still more preferably 3 to 60 min, further still more preferably 3 to 30 min, further still more preferably 3 to 20 min and further still more preferably 3 to 10 min. It is conceivable that due to the reaction time in the above range, active species for the subsequent oxidation reaction can be prepared industrially safely.

### [Oxidation step 2]

The method for producing furan-2,5-dicarboxylic acid of the present invention comprises, after the oxidation step 1, the oxidation step 2 in which 5-methylfurfural and an oxygen-containing gas are fed to the reaction container to carry out oxidation reaction under such a feeding condition that at least one of the oxygen concentration of the oxygen-containing gas and the molar ratio of oxygen in the oxygen-containing gas to 5-methylfurfural is higher than in the oxidation step 1.

The oxidation step 2 can provide furan-2,5-dicarboxylic acid in a high yield.

In the oxidation step 2, it is preferable that the lower aliphatic carboxylic acid, the bromine compound and the metal catalyst be mixed with 5-methylfurfural to be continuously fed. That is, the oxidation step 2 is preferably a step, after the oxidation step 1, of continuously feeding 5-methylfurfural, the lower aliphatic carboxylic acid, the bromine compound, the metal catalyst and the oxygen-containing gas to the reaction container to carry out oxidation reaction.

It is preferable that the amounts of the lower aliphatic carboxylic acid, the bromine compound and the metal catalyst be determined with respect to the whole 5-methylfurfural to be used in the present production method; and in the case of carrying out the oxidation step 2 continuously for a long period, it is preferable that these be mixed with 5-methylfurfural to be continuously fed, before feeding to the reaction container, so as to satisfy the preferable ratios thereof to the 5-methylfurfural.

It is conceivable that part of 5-methylfurfural can be turned to active species by oxidizing 5-methylfurfural under a relatively mild condition in the oxidation step 1, and in the subsequent present step, main oxidation reaction is carried out to obtain furan-2,5-dicarboxylic acid. Then, it is conceivable that in the oxidation step 2, the oxidation reaction can be progressed smoothly due to the higher oxygen concentration and/or the higher molar ratio of oxygen to the starting material than in the oxidation step 1. Hence, it is conceivable that stagnation of oxygen is not caused and that the highly safe production can be carried out. Further, it is conceivable that the starting material is evenly consumed during the reaction, resulting in suppression of side reactions, an excellent conversion rate, and an improved yield.

### <Oxygen-containing gas>

The oxygen-containing gas to be used in the present step is as described for the oxidation step 1. The oxygen concentration thereof is equal to or higher than that of the oxygen-containing gas used in the oxidation step 1.

Specifically, the oxygen-containing gas to be used in the present step is a mixed gas of oxygen and a gas other than oxygen. The gas other than oxygen to be mixed with oxygen is preferably an inert gas and more preferably nitrogen. The gas other than oxygen to be contained in the oxygen-containing gas in the oxidation step 2 is preferably a gas containing 95% by volume or higher of nitrogen and preferably a gas containing 98% by volume or higher of nitrogen.

As a gas containing nitrogen and oxygen, air is preferable.

Then, in order to adjust the oxygen concentration, oxygen gas or an oxygen-containing gas having a high oxygen concentration may be mixed with an inert gas such as nitrogen, before use.

For example, air having an oxygen concentration of 20.9% by volume can be used as it is. In the present step, it is preferable that the oxygen concentration be adjusted by air and nitrogen to obtain the oxygen-containing gas. From the safety and economical viewpoint, it is preferable to use air or a mixed gas of air and nitrogen as the oxygen-containing gas.

### [Condition and others of the oxidation step 2]

The present step is a step of continuously feeding 5-methylfurfural and an oxygen-containing gas to the reaction container to carry out oxidation reaction under such a feeding condition that at least one of the oxygen concentration of the oxygen-containing gas and the molar ratio of oxygen in the oxygen-containing gas to 5-methylfurfural is higher than in the oxidation step 1.

Here, "continuously feeding 5-methylfurfural and the oxygen-containing gas" means that the oxidation reaction of 5-methylfurfural in the reaction container and the feeding of 5-methylfurfural and the oxygen-containing gas are parallelly carried out, and that 5-methylfurfural and the oxygen-containing gas are fed for at least time longer than 50% of the reaction time during the reaction time from the initiation of the oxidation reaction to the end of the oxidation reaction. The feeding of 5-methylfurfural is carried out preferably for 60% or longer of the reaction time in the oxidation step 2, more preferably for 80% or longer thereof and still more preferably for 90% or longer thereof, and may be carried out for 100% of the reaction time in the oxidation step 2. The feeding of the oxygen-containing gas is preferably for 60% or longer of the reaction time in the oxidation step 2, more preferably for 80% or longer thereof and still more preferably for 90% or longer thereof, and may be carried out for 100% of the reaction time in the oxidation step 2.

When the feeding condition in the oxidation step 1 is the above feeding condition 1, in which the oxygen concentration is rather low, the oxygen concentration in the present step is preferably high. When the feeding condition in the oxidation step 1 is the above feeding condition 2, in which the molar ratio of oxygen to the starting material is rather low, the molar ratio of oxygen to the starting material in the present step is preferably high.

For example, preferably, the feeding condition in the oxidation step 1 be the feeding condition 1, and the oxygen concentration of the oxygen-containing gas in the oxidation step 2 is higher by 5% by volume or higher than that in the oxidation step 1. Alternatively, preferably, the feeding condition in the oxidation step 1 is the feeding condition 2, and the molar ratio of oxygen in the oxygen-containing gas to 5-methylfurfural in the oxidation step 2 is higher by 1.5 or higher than that in the oxidation step 1.

When the feeding condition in the oxidation step 1 is the feeding condition 1, the oxygen concentration of the oxygen-containing gas in the oxidation step 2 is higher than that in the oxidation step 1, preferably by 1% by volume or higher, preferably by 3% by volume or higher, preferably by 5% by volume or higher, preferably by 7% by volume or higher, or preferably by 8% by volume or higher. For the upper limit value, the oxygen concentration of the oxygen-containing gas in the oxidation step 2 is higher than the oxygen concentration of the oxygen-containing gas in the oxidation step 1, preferably by 20% by volume or lower, preferably by 15% by volume or lower, or preferably by 10% by volume or lower. Due to the oxygen concentration of the oxygen-containing gas in the above range, furan-2,5-dicarboxylic acid can be industrially highly safely obtained in a high conversion rate and a high yield.

When the feeding condition in the oxidation step 1 is the feeding condition 2, the molar ratio of oxygen in the oxygen-containing gas to 5-methylfurfural in the oxidation step 2 is higher than that in the oxidation step 1, preferably by 0.5 or higher, preferably by 1.0 or higher, preferably by 1.5 or higher, preferably by 1.6 or higher, or preferably by 1.8 or higher. For the upper limit value, the molar ratio of oxygen in the oxygen-containing gas to 5-methylfurfural in the oxidation step 2 is higher than the molar ratio of oxygen in the oxygen-containing gas to 5-methylfurfural in the oxidation step 1, preferably by 3.0 or lower, preferably by 2.5 or lower, or preferably by 2.0 or lower. Due to the molar ratio of oxygen in the oxygen-containing gas to 5-methylfurfural in the above range, furan-2,5-dicarboxylic acid can be industrially highly safely obtained in a high conversion rate and a high yield.

In the oxidation step 1, 5-methylfurfural and the oxygen-containing gas are fed under the feeding condition 1 or the feeding condition 2, and in either feeding condition case, the feeding condition of 5-methylfurfural and the oxygen-containing gas in the oxidation step 2 is preferably the following feeding condition 3.

The feeding condition 3: the oxygen concentration of the oxygen-containing gas is 15 to 30% by volume; and the molar ratio of oxygen in the oxygen-containing gas to 5-methylfurfural is 2.5 to 4.2.

When 5-methylfurfural and the oxygen-containing gas are fed under the feeding condition 3, the oxygen concentration of the oxygen-containing gas to be used is 15 to 30% by volume, and preferably 18 to 28% by volume, more preferably 20 to 25% by volume and still more preferably 20 to 22% by volume. Due to the oxygen concentration of the oxygen-containing gas in the above range, furan-2,5-dicarboxylic acid can be industrially highly safely obtained in a high conversion rate and a high yield.

When 5-methylfurfural and the oxygen-containing gas are fed under the feeding condition 3, the molar ratio of oxygen in the oxygen-containing gas to 5-methylfurfural is 2.5 to 4.2, and preferably 3.0 to 4.0, more preferably 3.1 to 3.8 and still more preferably 3.2 to 3.5. Due to the molar ratio of oxygen in the oxygen-containing gas to 5-methylfurfural in the above range, furan-2,5-dicarboxylic acid can be industrially highly safely obtained in a high conversion rate and a high yield.

The oxygen partial pressure in feeding of the oxygen-containing gas in the oxidation step 2 is preferably 0.05 to 0.2 MPa.

The reaction temperature in the oxidation step 2 is preferably 130 to 180°C.

Then, there is a preferable combination of the oxygen partial pressure and the reaction temperature. The preferable combination is specifically shown below.

The reaction temperature and the oxygen partial pressure in feeding of the oxygen-containing gas in the oxidation step 2 are preferably under the following condition 7 or the following condition 8.

The condition 7: the reaction temperature is 130°C or higher and lower than 160°C, and the oxygen partial pressure in feeding of the oxygen-containing gas is 0.15 to 0.2 MPa.

The condition 8: the reaction temperature is 160 to 180°C, and the oxygen partial pressure in feeding of the oxygen-containing gas is 0.11 MPa or higher and lower than 0.15 MPa.

The condition 7 is as follows: the reaction temperature is 130°C or higher and lower than 160°C, and the oxygen partial pressure in feeding of the oxygen-containing gas is 0.15 to 0.2 MPa.

In the case of feeding 5-methylfurfural and the oxygen-containing gas under the condition 7, the reaction temperature is preferably 130°C or higher and lower than 160°C, more preferably 135 to 155°C and still more preferably 140 to 150°C. Due to the reaction temperature in the above range, furan-2,5-dicarboxylic acid can be industrially highly safely obtained in a high conversion rate and a high yield.

In the case of feeding 5-methylfurfural and the oxygen-containing gas under the condition 7, the oxygen partial pressure in feeding of oxygen-containing gas is preferably 0.15 to 0.2 MPa. Due to the oxygen partial pressure in the above range, furan-2,5-dicarboxylic acid can be industrially highly safely obtained in a high conversion rate and a high yield.

The condition 8 is as follows: the reaction temperature is 160 to 180°C, and the oxygen partial pressure in feeding of the oxygen-containing gas is 0.11 MPa or higher and lower than 0.15 MPa.

In the case of feeding 5-methylfurfural and the oxygen-containing gas under the condition 8, the reaction temperature is preferably 160 to 180°C, more preferably 165 to 180°C and still more preferably 170 to 175°C. Due to the reaction temperature in the above range, furan-2,5-dicarboxylic acid can be industrially highly safely obtained in a high conversion rate and a high yield.

In the case of feeding 5-methylfurfural and the oxygen-containing gas under the condition 8, the oxygen partial pressure in feeding of the oxygen-containing gas is preferably 0.11 MPa or higher and lower than 0.15 MPa, more preferably 0.11 to 0.14 MPa and still more preferably 0.11 to 0.13 MPa. Due to the oxygen partial pressure in the above range, furan-2,5-dicarboxylic acid can be industrially highly safely obtained in a high conversion rate and a high yield.

The reaction time in the oxidation step 2 is not especially limited, and the reaction may be carried out until furan-2,5-dicarboxylic acid in a target amount (amount produced) can be obtained.

In particular, when the oxidation reaction is smoothly progressing, 5-methylfurfural and the lower aliphatic carboxylic acid can be continuously fed for a long period, whereby a large amount of furan-2,5-dicarboxylic acid can be obtained efficiently. Hence, the case is industrially preferable. It is more preferable that 5-methylfurfural, the lower aliphatic carboxylic acid, the bromine compound and the metal catalyst and the oxygen-containing gas be continuously fed over a long period.

Then, it is more preferable that furan-2,5-dicarboxylic acid obtained along with continuous feeding of 5-methylfurfural and the lower aliphatic carboxylic acid over a long period be appropriately discharged to be removed from the reaction system and collected. Discharging furan-2,5-dicarboxylic acid enables the reaction to be carried out in a longer period, which is industrially more preferable. It is still more preferable that furan-2,5-dicarboxylic acid obtained further be continuously discharged to be removed from the reaction system and collected. It is further still more preferable that 5-methylfurfural and the lower aliphatic carboxylic acid be continuously fed and that simultaneously, furan-2,5-dicarboxylic acid obtained be continuously discharged to be removed from the reaction system and collected.

It is further still more preferable that 5-methylfurfural, the lower aliphatic carboxylic acid, the bromine compound, the metal catalyst and the oxygen-containing gas be continuously fed over a long period, and that furan-2,5-dicarboxylic acid obtained be discharged. It is further still more preferable that 5-methylfurfural, the lower aliphatic carboxylic acid, the bromine compound, the metal catalyst and the oxygen-containing gas be continuously fed over a long period, and that furan-2,5-dicarboxylic acid obtained be continuously discharged. It is further still more preferable that 5-methylfurfural, the lower aliphatic carboxylic acid, the bromine compound, the metal catalyst and the oxygen-containing gas be continuously fed over a long period, and that simultaneously, furan-2,5-dicarboxylic acid obtained be continuously discharged.

It is preferable that the amounts of the lower aliphatic carboxylic acid, the bromine compound and the metal catalyst to be fed herein be determined with respect to the whole 5-methylfurfural to be used in the present production method; and in the case of carrying out the oxidation step 2 continuously for a long period, it is preferable that components shown below be mixed with 5-methylfurfural before feeding to the reaction container such that the ratio (amount) of each component to the 5-methylfurfural to be continuously fed satisfies the preferable ratio (amount) of each component, which will be shown above.

In the present step, 5-methylfurfural and the oxygen-containing gas are continuously fed to the reaction mixture having been subjected to the oxidation step 1, as described above. Preferably, 5-methylfurfural, the lower aliphatic carboxylic acid, the bromine compound, the metal catalyst and the oxygen-containing gas are continuously fed to the reaction mixture having been subjected to the oxidation step 1, as described above.

It is simple and therefore preferable that each component to be used in the present step be the same as in the oxidation step 1.

### [Other steps]

The method for producing furan-2,5-dicarboxylic acid of the present invention may include an optional step in addition to the oxidation step 1 and the oxidation step 2.

Examples of the optional step included in the method for producing furan-2,5-dicarboxylic acid of the present invention includes the solvent removal step and the purification step.

The solvent removal step is the step of removing the lower aliphatic carboxylic acid and water, which are solvents having low boiling points, from a product containing furan-2,5-dicarboxylic acid as the target material of the present production method. Removal of these solvents before the subsequent purification step can lead to favorable efficiency in the subsequent purification step, in which by-products formed by the oxidation reaction and 5-methylfurfural as the starting material are removed.

In the solvent removal step, the solvents may be distilled off by vacuum heat distillation or by atmospheric heat distillation, in view of efficiently removing the solvents. Alternatively, the solvents may be removed by the following purification step.

The purification step is carried out by any method as long as the method can separate and collect furan-2,5-dicarboxylic acid as the target material in a high purity, and may include, for example, reprecipitation or recrystallization.

Further, hydrogenation may be carried out to remove coloring components as a byproduct produced in the oxidation reaction.

### Examples

The present invention will be described specifically by way of Examples shown below, but the present invention is not any more limited to these Examples.

### [Evaluations]

### <Exit oxygen concentration>

The exit oxygen concentration was determined as follows.
Apparatus: manufactured by SHIMADZU CORPORATION, portable oximeter: POT-101

Measurement method: a gas discharge pipe of an autoclave was connected to the oximeter, and the exit oxygen concentration was measured in real time and evaluated based on the following criteria.

Here, it is preferable in view of safety in industrial production that the exit oxygen concentration be not higher than 10% by volume, which is the explosion limit of acetic acid as a solvent, and a lower exit oxygen concentration is preferable because of a higher freedom of the reaction condition from a safety standpoint. In Examples and Comparative Examples, the evaluation was made relatively to a concentration of 8% by volume as a reference, which did not reach the explosion limit even in sharp fluctuation in the concentration.

For Comparative Examples in which the exit oxygen concentration exceeded 8% by volume, a time (min) from the initiation of the oxidation reaction to the time point when the exit oxygen concentration exceeded 8% by volume is shown in Table 1.

### (Evaluation criteria)

A: The exit oxygen concentration was always 8% by volume or lower during the oxidation reaction.
B: The exit oxygen concentration exceeded 8% by volume during the oxidation reaction.

### <5-Methylfurfural conversion rate>

The amount (mol) of 5-methylfurfural consumed was determined by calculating the amount (mol) of 5-methylfurfural contained in an oxidation reaction product after the oxidation step 2 by an internal standard method (internal standard: triphenylmethane) using gas chromatography, and subtracting the calculated result from the amount (mol) of 5-methylfurfural as the starting material.

The conversion rate was determined by the following expression from the above amount of 5-methylfurfural consumed and the above amount of 5-methylfurfural as the starting material. Here, the conversion rate was a starting material conversion rate.

5-Methylfurfural conversion rate (%) = (amount (mol) of 5-methylfurfural consumed)/(amount (mol) of 5-methylfurfural as the starting material) × 100

A higher 5-methylfurfural conversion rate is preferable because it means that the starting material can be more efficiently converted to furan-2,5-dicarboxylic acid as a product.

### <Furan-2,5-dicarboxylic acid yield>

The amount (mol) of furan-2,5-dicarboxylic acid (amount of furan-2,5-dicarboxylic acid formed) contained in an oxidation reaction product after the oxidation step 2 was calculated by an internal standard method (internal standard: triphenylmethane) using gas chromatography.

The furan-2,5-dicarboxylic acid selectivity was determined by the following expression from the above-mentioned amount of furan-2,5-dicarboxylic acid formed and the amount of 5-methylfurfural (amount of 5-methylfurfural consumed) as the starting material.

Furan-2,5-dicarboxylic acid selectivity (%) = (an amount (mol) of furan-2,5-dicarboxylic acid formed)/(an amount (mol) of 5-methylfurfural consumed) × 100

Then, the furan-2,5-dicarboxylic acid yield is a value calculated as a product of the conversion rate and the furan-2,5-dicarboxylic acid selectivity. Higher furan-2,5-dicarboxylic acid selectivity and furan-2,5-dicarboxylic acid yield are preferable because they means that a high-purity furan-2,5-dicarboxylic acid can be obtained more efficiently.

### <Amount of high molecular weight substances>

A gas discharge pipe of an autoclave was connected to a portable gas concentration measuring device (CGT-7100, manufactured by SHIMADZU CORPORATION), and the amount of a carbon-containing gas (carbon monoxide and carbon dioxide) discharged was measured. The amount of carbon of the carbon-containing gas was calculated from the found value of the above amount of the carbon-containing gas. Also, the amount of carbon of 5-methylfurfural of the starting material and the amount of carbon of furan-2,5-dicarboxylic acid in the product were calculated and determined.

The proportion obtained by the following expression was regarded as the amount of high molecular weight substances in the product. Here, the amounts of carbon shown below were all in terms of moles. A smaller amount of the high molecular weight substances is preferable because it means that the purity of furan-2,5-dicarboxylic acid obtained was excellent.

The amount (% by mol) of the high molecular weight substances = (amount of carbon of 5-methylfurfural of the starting material - amount of carbon of the carbon-containing gas - amount of carbon of furan-2,5-dicarboxylic acid in the product)/(amount of carbon of 5-methylfurfural of the starting material) × 100

### Example 1 (Production of furan-2,5-dicarboxylic acid)

### (1. Oxidation step 1)

Cobalt acetate tetrahydrate, manganese acetate tetrahydrate, a 48 mass% hydrogen bromide aqueous solution, glacial acetic acid and water were mixed such that the concentrations of cobalt metal atoms, manganese metal atoms, bromine ions, acetic acid and water were values indicated in Table 1, to thereby obtain a catalyst liquid.

In a 500 mL-internal volume titanium-made autoclave having a gas discharge pipe with a reflux condenser, a gas blowing pipe, a continuous feed pump for the starting material, and a stirrer, 120 g of the catalyst liquid was charged, and, under a nitrogen atmosphere, the temperature and the pressure were raised to 140°C and 1.0 MPa, respectively.

Then, the catalyst liquid and 5-methylfurfural were mixed in a mass ratio of the catalyst liquid/5-methylfurfural = 13/5 (72/28) to thereby obtain a starting material liquid.

The feeding of the starting material liquid to a reactor and the feeding of an air diluted with nitrogen (oxygen concentration: 12% by volume) to a reactor were simultaneously initiated, and the starting material liquid and the air diluted with nitrogen were continuously fed for 42 min to carry out an oxidation step 1. The condition in the oxidation step 1 is indicated in Table 1. The molar ratio of oxygen in the oxygen-containing gas to 5-methylfurfural was a value calculated from the amount of 5-methylfurfural contained in the starting material liquid and the amount of oxygen in the oxygen-containing gas fed simultaneously with the starting material liquid. Here, the oxygen partial pressure was a value calculated from the oxygen concentration of the oxygen-containing gas and the vapor pressure of acetic acid.

### (2. Oxidation step 2)

After the finish of the oxidation step 1, an oxidation step 2 was carried out by using air (oxygen concentration: 20.9% by volume) in place of the air diluted with nitrogen (oxygen concentration: 12% by volume) under the condition indicated in Table 1 to thereby obtain a product containing furan-2,5-dicarboxylic acid. The starting material liquid and air were continuously fed for 129 min. Here, during the oxidation step 1 and the oxidation step 2, the exit oxygen concentration never exceeded 8% by volume. Table 1 shows the 5-methylfurfural conversion rate, the furan-2,5-dicarboxylic acid yield and the amount of high molecular weight substances of the obtained product. Here, the total amount of 5-methylfurfural resultantly fed is indicated as "total amount of 5-methylfurfural" in Table 1.

### Examples 2 and 3 and Comparative Examples 1 to 7 (production of furan-2,5-dicarboxylic acid)

Products containing furan-2,5-dicarboxylic acid were obtained in the same manner as in Example 1, except for changing the conditions of the oxidation steps to conditions indicated in Table 1. In any case, a starting material liquid and air or an air diluted with nitrogen were continuously fed.

In Example 3, the oxygen-containing gas was fed for initial 35 min of the oxidation step 2 such that the molar ratio of oxygen in the oxygen-containing gas to 5-methylfurfural was 3.4; thereafter, the amount of air fed was increased, and specifically, the oxygen-containing gas was fed for remaining 122 min such that the molar ratio of oxygen in the oxygen-containing gas to 5-methylfurfural to was 4.0. In Comparative Examples 1 to 4 and 6, the oxidation step was carried out under a single condition from the initiation to the end. Table 1 shows the 5-methylfurfural conversion rate, the furan-2,5-dicarboxylic acid yield and the amount of high molecular weight substances of the obtained product.

### [Table 1]

**Table 1**

| | | | Example 1 | Example 2 | Example 3 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|---|
| Catalyst Liquid | Concentration of cobalt metal atoms | % by mass | 0.36 | 0.37 | 0.46 | 0.23 | 0.23 | 0.23 | 0.23 | 0.46 | 0.23 | 0.23 |
| | Concentration of manganese metal atoms | % by mass | 0.039 | 0.039 | 0.049 | 0.024 | 0.024 | 0.024 | 0.024 | 0.049 | 0.021 | 0.023 |
| | Concentration of bromine ions | % by mass | 0.52 | 0.52 | 0.65 | 0.33 | 0.33 | 0.33 | 0.33 | 0.65 | 0.31 | 0.32 |
| | Concentration of water | % by mass | 5.6 | 5.6 | 7.5 | 5.6 | 5.6 | 5.6 | 5.6 | 7.5 | 5.7 | 5.6 |
| | Concentration of acetic acid | % by mass | 93.481 | 93.468 | 91.337 | 93.819 | 93.819 | 93.819 | 93.819 | 91.337 | 93.737 | 93.826 |
| Starting material liquid | (Catalyst liquid/5-methylfurfural) | mass ratio | 72/28 | 72/28 | 72/28 | 82/18 | 82/18 | 82/18 | 82/18 | 72/28 | 77/23 | 79/21 |
| Oxidation Step 1 | Temperature | °C | 140 | 140 | 173 | - | - | - | - | 173 | - | 140 |
| | Feed rate of starting material | g/min | 0.71 | 0.93 | 1.1 | - | - | - | - | 0.94 | - | 1.5 |
| | Feed rate of 5-methylfurfural | g/min | 0.1988 | 0.2604 | 0.308 | - | - | - | - | 0.2632 | - | 0.315 |
| | Feed rate of 5-methylfurfural | mol/min | 0.0018 | 0.0024 | 0.0028 | - | - | - | - | 0.0024 | - | 0.0029 |
| | Concentration of oxygen in oxygen-containing gas | % by volume | 12 | 20.9 | 12 | - | - | - | - | 12 | - | 12 |
| | Oxygen partial pressure | MPa | 0.106 | 0.184 | 0.07 | - | - | - | - | 0.07 | - | 0.106 |
| | Molar ratio of oxygen in oxygen-containing gas to 5-methylfurfural | | 3.7 | 1.8 | 3.4 | - | - | - | - | 4.7 | - | 4.5 |
| | Reaction time of oxidation step 1 | min | 42 | 17 | 6 | - | - | - | - | 6 | - | 30 |
| Oxidation Step 2 | Temperature | °C | 140 | 140 | 173 | 140 | 140 | 140 | 140 | 173 | 140 | 140 |
| | Pressure | MPaG | 1.0 | 1.0 | 1.0 | 1.0 | 1.8 | 1.0 | 1.0 | 1.0 | 1.0 | 1.0 |
| | Feed rate of starting material | g/min | 1.18 | 0.93 | 1.1 | 1.57 | 1.5 | 1.64 | 1.64 | 0.94 | 1.5 | 1.5 |
| | Feed rate of 5-methylfurfural | g/min | 0.33 | 0.26 | 0.31 | 0.28 | 0.28 | 0.30 | 0.30 | 0.26 | 0.35 | 0.32 |
| | Feed rate of 5-methylfurfural | mol/min | 0.0030 | 0.0024 | 0.0028 | 0.0026 | 0.0025 | 0.0027 | 0.0027 | 0.0024 | 0.0031 | 0.0029 |
| | Concentration of oxygen in oxygen-containing gas | % by volume | 20.9 | 20.9 | 20.9 | 4 | 20.9 | 20.9 | 20.9 | 20.9 | 20.9 | 20.9 |
| | Oxygen partial pressure | MPa | 0.184 | 0.184 | 0.121 | 0.035 | 0.4 | 0.18 | 0.180 | 0.120 | 0.184 | 0.184 |
| | Molar ratio of oxygen in oxygen-containing gas to 5-methylfurfural | | 3.7 | 3.7 | 3.4, 4.0 | 0.67 | 4.0 | 1.69 | 0.85 | 4.7 | 3.7 | 4.5 |
| | Reaction time of oxidation step 2 | min | 129 | 175 | 157(35, 122) | 115 | 120 | 108 | 108 | 186 | 31.8 | 12 |
| Total Reaction Time | | min | 171 | 192 | 163 | 115 | 120 | 108 | 108 | 192 | 31.8 | 42 |
| Total Amount of 5-methylfurfural | | g | 50 | 50 | 50 | 33 | 33 | 33 | 33 | 50 | 9 | 10 |
| Evaluations | Exit oxygen concentration | | A | A | A | A | B | B | A | B | B | B |
| | Time point when exit oxygen concentration exceeded 8% | min | - | - | - | - | 15 | 60.7 | - | 14 | 31.8 | 42 |
| | 5-Methylfurfural conversion rate | % by mol | 99.4 | 99.4 | 100.0 | 35.5 | 99.5 | 47.0 | 73.8 | 100.0 | 61.9 | 97.8 |
| | Furan-2,5-dicarboxylic acid yield | % by mol | 70.1 | 70.2 | 70.5 | 8.5 | 39.9 | 9.3 | 8.7 | 60.0 | 2.9 | 61.5 |
| | Amount of high molecular weight substances | % by mol | 11.0 | 12.0 | 8.3 | 39.9 | 39.9 | 1.0 | 41.7 | 14.0 | 1.5 | 30.6 |

As shown in Table 1, it is clear that according to the production methods of Examples, furan-2,5-dicarboxylic acid was obtained in a high yield, and that the conversion rate from 5-methylfurfural as the starting material was also excellent. It is also clear that it was possible to carry out the oxidation reaction while the rise in the exit oxygen concentration (oxygen concentration of off-gas) was suppressed.

Therefore, it is clear that according to the method for producing furan-2,5-dicarboxylic acid of the present invention, furan-2,5-dicarboxylic acid can be obtained in a high yield and also the excellent conversion rate. It is also clear that the method for producing furan-2,5-dicarboxylic acid is industrially highly safety.

## Claims

1. A method for producing furan-2,5-dicarboxylic acid, comprising:
an oxidation step 1 of continuously feeding 5-methylfurfural and an oxygen-containing gas in the presence of a lower aliphatic carboxylic acid, a bromine compound and a metal catalyst to a reaction container to carry out oxidation reaction under the following feeding condition 1 or the following feeding condition 2; wherein the bromine compound is at least one selected from the group consisting of hydrogen bromide and bromide salts, and the metal catalyst is at least one selected from the group consisting of cobalt catalysts and manganese catalysts, and
an oxidation step 2, after the oxidation step 1, of continuously feeding 5-methylfurfural and an oxygen-containing gas to the reaction container to carry out oxidation reaction under such a feeding condition that at least one of an oxygen concentration of the oxygen-containing gas and a molar ratio of oxygen in the oxygen-containing gas to 5-methylfurfural is higher than in the oxidation step 1:
the feeding condition 1: the oxygen concentration of the oxygen-containing gas is 10% by volume or higher and lower than 15% by volume, and the molar ratio of oxygen in the oxygen-containing gas to 5-methylfurfural is 2.5 to 4.2; and
the feeding condition 2: the oxygen concentration of the oxygen-containing gas is 15 to 30% by volume, and the molar ratio of oxygen in the oxygen-containing gas to 5-methylfurfural is 1.0 or higher and lower than 2.5.

2. The method for producing furan-2,5-dicarboxylic acid according to claim 1, wherein
the feeding condition in the oxidation step 1 is the feeding condition 1, and the oxygen concentration of the oxygen-containing gas in the oxidation step 2 is higher by 5% by volume or higher than the oxygen concentration of the oxygen-containing gas in the oxidation step 1; or
the feeding condition in the oxidation step 1 is the feeding condition 2, and the molar ratio of oxygen in the oxygen-containing gas to 5-methylfurfural in the oxidation step 2 is higher by 1.5 or higher than the molar ratio of oxygen in the oxygen-containing gas to 5-methylfurfural in the oxidation step 1.

3. The method for producing furan-2,5-dicarboxylic acid according to claim 1 or 2, wherein the feeding condition of 5-methylfurfural and the oxygen-containing gas in the oxidation step 2 is the following feeding condition 3:
the feeding condition 3: the oxygen concentration of the oxygen-containing gas is 15 to 30% by volume, and the molar ratio of oxygen in the oxygen-containing gas to 5-methylfurfural is 2.5 to 4.2.

4. The method for producing furan-2,5-dicarboxylic acid according to claim 1 or 2, wherein the feeding condition of 5-methylfurfural and the oxygen-containing gas in the oxidation step 1 is the following feeding condition 4:
the feeding condition 4: a product of the oxygen concentration (volume) of the oxygen-containing gas and the molar ratio of oxygen in the oxygen-containing gas to 5-methylfurfural is 0.3 to 0.5.

5. The method for producing furan-2,5-dicarboxylic acid according to claim 1 or 2, wherein an oxygen partial pressure in feeding of the oxygen-containing gas in the oxidation step 1 is 0.05 to 0.2 MPa.

6. The method for producing furan-2,5-dicarboxylic acid according to claim 1 or 2, wherein a reaction temperature in the oxidation step 1 is 130 to 180°C.

7. The method for producing furan-2,5-dicarboxylic acid according to claim 1 or 2, wherein the reaction temperature and the oxygen partial pressure in feeding of the oxygen-containing gas in the oxidation step 1 are under the following condition 5 or the following condition 6:
the condition 5: the reaction temperature is 130°C or higher and lower than 160°C, and the oxygen partial pressure in feeding of the oxygen-containing gas is 0.10 to 0.2 MPa; and
the condition 6: the reaction temperature is 160 to 180°C, and the oxygen partial pressure in feeding of the oxygen-containing gas is 0.05 MPa or higher and lower than 0.10 MPa.

8. The method for producing furan-2,5-dicarboxylic acid according to claim 1 or 2, wherein an oxygen partial pressure in feeding of the oxygen-containing gas in the oxidation step 2 is 0.05 to 0.2 MPa.

9. The method for producing furan-2,5-dicarboxylic acid according to claim 1 or 2, wherein a reaction temperature in the oxidation step 2 is 130 to 180°C.

10. The method for producing furan-2,5-dicarboxylic acid according to claim 1 or 2, wherein the reaction temperature and the oxygen partial pressure in feeding of the oxygen-containing gas in the oxidation step 2 are under the following condition 7 or the following condition 8:
the condition 7: the reaction temperature is 130°C or higher and lower than 160°C, and the oxygen partial pressure in feeding of the oxygen-containing gas is 0.15 to 0.2 MPa; and
the condition 8: the reaction temperature is 160 to 180°C, and the oxygen partial pressure in feeding of the oxygen-containing gas is 0.11 MPa or higher and lower than 0.15 MPa.

11. The method for producing furan-2,5-dicarboxylic acid according to claim 1 or 2, wherein a reaction time in the oxidation step 1 is 3 to 200 min.

12. The method for producing furan-2,5-dicarboxylic acid according to claim 1 or 2, wherein the metal catalyst is at least one selected from the group consisting of aliphatic carboxylates of cobalt and aliphatic carboxylates of manganese.

13. The method for producing furan-2,5-dicarboxylic acid according to claim 1 or 2, wherein the lower aliphatic carboxylic acid is acetic acid.

14. The method for producing furan-2,5-dicarboxylic acid according to claim 1 or 2, wherein the bromine compound is at least one selected from the group consisting of hydrogen bromide, sodium bromide, potassium bromide and ammonium bromide.

15. The method for producing furan-2,5-dicarboxylic acid according to claim 1 or 2, wherein a gas other than oxygen contained in the oxygen-containing gas in the oxidation step 1 contains 95% by volume or higher of nitrogen.

16. The method for producing furan-2,5-dicarboxylic acid according to claim 1 or 2, wherein a gas other than oxygen contained in the oxygen-containing gas in the oxidation step 2 contains 95% by volume or higher of nitrogen.
